# EUROPEAN PATENT SPECIFICATION

(11) **EP 4 436 466 B1**
(45) Date of publication and mention of the grant of the patent: **17.06.2026**
(21) Application number: 22895746.0
(22) Date of filing: 25.01.2022
(51) Int. Cl.: A61B 5/00, A61B 5/053

(54) **SYSTEM AND METHODS FOR PROVIDING INFORMATION FOR DIAGNOSING PRETERM BIRTH RISK**
SYSTEM UND VERFAHREN ZUR BEREITSTELLUNG VON INFORMATIONEN ZUR DIAGNOSE DES RISIKOS EINER FRÜHGEBURT
SYSTÈME ET PROCÉDÉS DE FOURNITURE D'INFORMATIONS POUR DIAGNOSTIQUER UN RISQUE DE NAISSANCE PRÉMATURÉE

(30) Priority: 22.11.2021 US 202117532715
(43) Date of publication of application: 02.10.2024
(73) Proprietor: The University Of Sheffield, Sheffield South, Yorkshire S10 2TN (GB); Sheffield Teaching Hospitals NHS Foundation Trust, Sheffield, South Yorkshire S10 2SB (GB)
(72) Inventor: SCARTH, Gordon, Prairie, MN 53346 (US); MONDRY, Jack, Minneapolis, MN 55435 (US); PARK, Byunggaun, Seoul 06628 (KR); GONG, Hyunseon, Seoul 06628 (KR); HEALEY, Timothy James, Sheffield, South Yorkshire (GB); ANUMBA, Dilichukwu, Sheffield, South Yorkshire (GB)
(74) Representative: HGF
(86) International application number: PCT/KR2022/001286
(87) International publication number: WO 2023/090533

(56) References cited:
- WO-A1-2017/109481
- JP-A- 2006 525 099
- JP-A- H07 184 923
- US-A1- 2011 313 311
- US-A1- 2017 035 347
- US-B1- 6 845 264
- JOKHI ROOBIN P ET AL: "Reproducibility and repeatability of measuring the electrical impedance of the pregnant human cervix-the effect of probe size and applied pressure", BIOMEDICAL ENGINEERING ONLINE, BIOMED CENTRAL LTD, LONDON, GB, vol. 8, no. 1, 17 June 2009 (2009-06-17), pages 10, XP021058634, ISSN: 1475-925X, DOI: 10.1186/1475-925X-8-10

## Description

### Technical Field

### CROSS REFERENCE TO RELATED APPLICATIONS

This application claims the benefit of U.S. patent application Serial No. 17/532,715, filed November 22, 2021.

This disclosure relates to a system for providing information for diagnosing preterm birth risk, more specifically a system for providing information for diagnosing preterm birth risk by measuring biomarkers (e.g., electrical impedances) of the human cervical tissues.

### Background Art

Preterm birth is a significant problem and causes hazardous health problems to infants and mothers. Early diagnosis and treatment of preterm birth can aid in delaying the delivery of the child by applying effective treatment to the mothers. It has been suggested that electrical impedance spectroscopy (EIS) techniques might be used for detecting the preterm birth. Details for the EIS techniques can be explained with the reference. For example, U.S. patent application Ser. No. 16/065,209 titled "Apparatus and methods for determining force applied to the tip of a probe" filed on Dec. 21, 2016, the entire disclosure of which is hereby incorporated by reference, for all purposes, as if fully set forth herein.

Document WO 2017/109481A1 discloses an apparatus capable of determining the force applied to the tip of an electrical impedance spectroscopy probe.

### Summary

Embodiments of the invention are set out in the appended claims. The description and drawings may also present additional non-claimed embodiments, exemplary embodiments, examples, aspects and implementations for the better understanding of the claimed embodiments defined in the appended claims.

### Disclosure of Invention

### Solution to Problem

One or more embodiments of the present disclosure can detect data including the impedance of cervical tissues at various frequencies, and provide information for predicting the preterm birth from the data. The embodiments presented herein also provide early diagnosis and treatment for preterm birth.

### Brief Description of Drawings

FIG. 1A illustrates a block diagram illustrating an apparatus or a system for providing information for diagnosing preterm birth risk with a cervix according to some embodiments of the present disclosure.
FIG. 1B illustrate a conceptual diagram illustrating an example of a diagnosis engine according to some embodiments of the present disclosure.
FIGs. 2A to 2D illustrate an example of a measuring device according to some embodiments of the present disclosure.
FIGs.2E and 2F illustrate an example of a contact mechanism according to some embodiments of the present disclosure.
FIGs. 2G and 2H illustrate
FIG. 3 illustrates an example of a disposable tip according to some embodiments of the present disclosure.
FIGs. 4A, 4C, and 4E illustrate attachment/detachment mechanisms of a disposable tip and a measuring device according to some embodiments of the present disclosure.
FIGs. 4B, 4D, and 4F illustrate an enlarged view of the portion Z of FIGs. 4A, 4C, and 4E, respectively.
FIG. 5 illustrates a block diagram illustrating an apparatus or a system for providing information for diagnosing preterm birth risk according to some embodiments of the present disclosure.
FIG. 6 illustrate an example of a cervix.
FIGs. 7A and 7B illustrate an enlarged view of the portion A of FIG. 6.
FIGs. 8A and 8B illustrate a graph of mean cervical impedances of mothers who experienced preterm delivery and term delivery.
FIGs. 9A and 9B illustrate a flow-chart explaining a method of providing information for diagnosing preterm birth risk according to an embodiment of the present disclosure.
FIG. 10 illustrates an example of calibrating a disposable tip according to an embodiment of the present disclosure.
FIG. 11 illustrates an example of checking data requirements according to an embodiment of the present disclosure.

### Mode for the Invention

It is to be understood that the disclosure herein is not intended to limit the scope to the described embodiments, but includes various modifications, equivalents, and/or alternatives of the embodiments. In the description of the drawings, like reference numerals refer to like elements throughout the description of drawings.

Conditional language used herein, such as, among others, "can," "could," "might," "may," *"e.g.,"* and the like, unless specifically stated otherwise, or otherwise understood within the context as used, is generally intended to convey that certain embodiments include, while other embodiments do not include, certain features, elements and/or steps. Thus, such conditional language is not generally intended to imply that features, elements and/or steps are in any way selected (or required) for one or more embodiments or that one or more embodiments necessarily include logic for deciding, with or without author input or prompting, whether these features, elements and/or steps are included or are to be performed in any particular embodiment. The terms "comprising," "including," "having," and the like are synonymous and are used inclusively, in an open-ended fashion, and do not exclude additional elements, features, acts, operations, and so forth. Also, the term "or" is used in its inclusive sense (and not in its exclusive sense) so that when used, for example, to connect a list of elements, the term "or" means one, some, or all of the elements in the list. In addition, the articles "a," "an," and "the" as used in this application and the appended claims are to be construed to mean "one or more" or "at least one" unless specified otherwise. Except as specifically defined herein, all technical and scientific terms used herein are to be given as broad a commonly understood meaning as possible while maintaining claim validity.

As used herein, a phrase referring to "at least one of" a list of items refers to any combination of those items, including single members. As an example, "at least one of: A, B, or C" is intended to cover: A, B, C, A and B, A and C, B and C, and A, B, and C. Conjunctive language such as the phrase "at least one of X, Y and Z," unless specifically stated otherwise, is otherwise understood with the context as used in general to convey that an item, term, etc., may be at least one of X, Y or Z. Thus, such conjunctive language is not generally intended to imply that certain embodiments require at least one of X, at least one of Y and at least one of Z to each be present.

Similarly, while operations may be depicted in the drawings in a particular order, it is to be recognized that such operations need not be performed in the particular order shown or in sequential order, or that all illustrated operations be performed, to achieve beneficial results. Further, the drawings may schematically depict one more example processes in the form of a flowchart. However, other operations that are not depicted can be incorporated in the example methods and processes that are schematically illustrated. For example, one or more additional operations can be performed before, after, simultaneously, or between any of the illustrated operations. Additionally, the operations may be rearranged or reordered in other implementations. In certain circumstances, multitasking and parallel processing may be advantageous. Moreover, the separation of various system components in the implementations described above should not be understood as requiring such separation in all implementations, and it should be understood that the described program components and systems can generally be integrated together in a single software product or packaged into multiple software products. Additionally, other implementations are within the scope of the following claims. In some cases, the actions recited in the claims can be performed in a different order and still achieve beneficial results.

Terms such as "first" and "second" may be used to modify various elements regardless of order and/or importance. Those terms are only used for the purpose of differentiating a component from other components.

When an element (*e.g.,* a first constituent element) is referred to as being "operatively or communicatively coupled to" or "connected to" another element (*e.g.,* a second constituent element), it should be understood that each constituent element is directly connected or indirectly connected via another constituent element (*e.g.,* a third constituent element). However, when an element (*e.g.,* a first constituent element) is referred to as being "directly coupled to" or "directly connected to" another element ( *e.g.,* a second constituent element), it should be understood that there is no other constituent element (*e.g.,* a third constituent element) interposed therebetween.

The expression "configured to" as used in the present disclosure can refer to, for example, "suitable for," "having the capacity to," "designed to," "adapted to," "made to," or "capable of" depending on the situation. The term "configured to (or set to)" may not necessarily mean "specifically designed to" in hardware. Instead, in some circumstances, the expression "a device configured to" may mean that the device "is able to~" with other devices or components. For example, "a sub-processor configured to (or set to) execute A, B, and C" may be implemented as a processor dedicated to performing the operation (*e.g*., an embedded processor), or a generic-purpose processor (*e.g.,* a central processor unit (CPU) or an application processor) that can perform the corresponding operations.

Hereinafter, the present disclosure will be described in detail with reference to the accompanying drawings. Embodiments will be described in detail with reference to the accompanying drawings so that those skilled in the art can easily embody the present disclosure. The present disclosure may, however, be embodied in many different forms and should not be construed as limited to the embodiments set forth herein. In order to clearly illustrate the present disclosure, parts not related to the description are omitted, and like elements are denoted by like reference numerals throughout the specification.

### Example of an Apparatus or a System

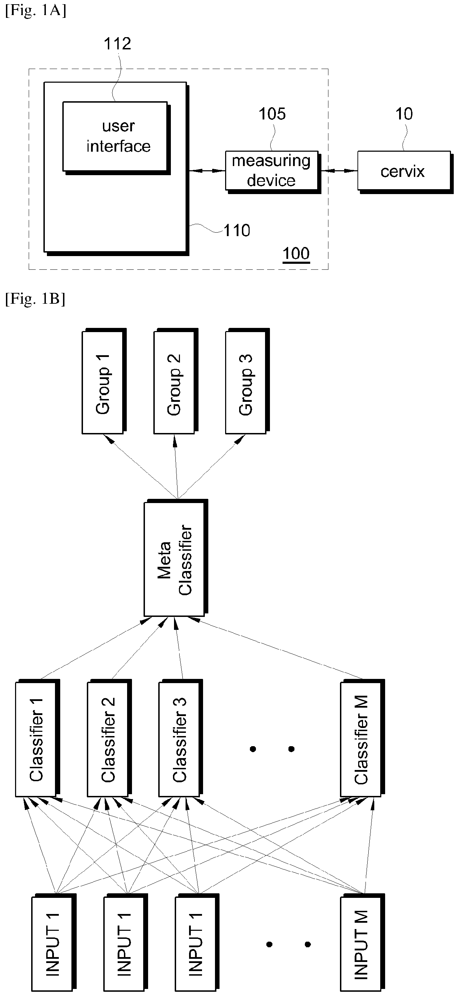

FIG. 1 illustrates a block diagram illustrating an apparatus or a system 100 for providing information for diagnosing preterm birth risk with a cervix 10 according to some embodiments of the present disclosure. Within the context of the present disclosure, the two terms, "system" and "apparatus", refer to the same concept and are used interchangeably.

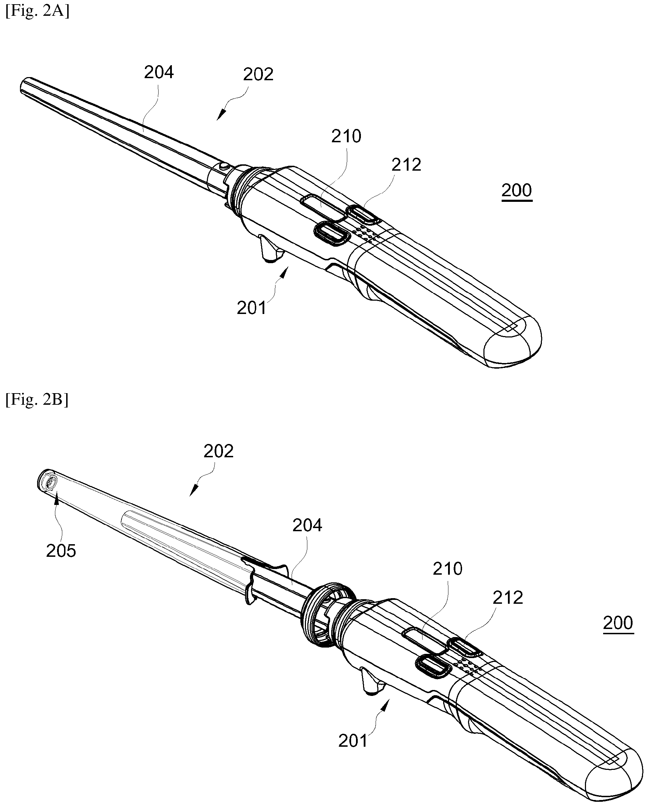

The system 100 may comprise a measuring device 105 and an information provider 110 including a user interface 112. The system 100 is configured to receive data including impedances from the cervix 10. In an embodiment, the measuring device may include a probe tip portion (202, Fig. 2A), which are electrically coupled to tissues of the cervix 10 via electrodes (*e.g.,* 206 transmit electrodes, detect electrodes, Fig. 2B). An electrical path is established between the measuring device 105 and the cervical tissues.

The measuring device 105 may generate and apply a plurality of signals (*e.g*., sinusoidal, square wave, triangular wave, saw-toothed wave, etc.), for example, current or voltage, at different frequencies sequentially and/or concurrently to the cervix 10, for example, to the target cervical tissues. The measuring device 105 receives data including a plurality of signals (*e.g.*, sinusoidal, square wave, triangular wave, saw-toothed wave, etc.) from the cervix 10. The measuring device 105 may obtain impedances corresponding the cervical tissue based on the plurality of signals from the cervix 10. The measuring device 105 transfer the impedances to the information provider 110.

In an embodiment, the term, sinusoidal signals, will be used only for explanation purpose. The plurality of sinusoidal signals applied to the cervical tissues may be called as a plurality of first sinusoidal signals. A plurality of sinusoidal signals received by measuring device 105 may be called as a plurality of second sinusoidal signals. The impedances may be calculated from the first and the second sinusoidal signals at difference frequencies, respectively.

In an embodiment, the first sinusoidal signals are injected into the cervical tissues, traversed through the cervical tissues, and detected as the second sinusoidal signals. The first sinusoidal signals are attenuated by the impedance of the cervical tissues. The first sinusoidal signals may have a frequency as same as that of the second sinusoidal frequency but with a different amplitude and phase.

The information provider 110 is configured to output information for diagnosing preterm birth risk based on correlation between the plurality of corresponding impedances and the preterm birth risk. In an embodiment, the information provider 110 is configured to output information for diagnosing preterm birth risk based on correlation between the plurality of corresponding impedances. The information provider 110 can classify the data into different groups, each representing a different preterm risk state or risk level. The user interface 112 is configured to notify a user of the information for diagnosing preterm birth risk. The user interface 112 may comprise at least one of a display for showing numerical values, a speaker, and a visual indicator such as LEDs for providing different colors, each indicating different preterm risk states.

In an embodiment, the information provider 110 may output information for diagnosing preterm birth risk based on correlation between the plurality of corresponding impedances, another data, and the preterm birth risk. Said another data may be at least one of spectral impedances, pH level, metabolites, temperature, pregnancy stage, and human microbiome. In an embodiment, the spectral impedances include impedances at different frequencies.

The information provider 110 may comprise a diagnosis engine. The diagnosis engine may comprise an algorithm, an AI (Artificial Intelligence) model, or a machine learning model. Within the context of the present disclosure, these terms (algorithm, AI model, machine learning model) can be used for referring to the same concept. The diagnosis engine may be trained with scientific discipline that is concerned with the design and development of algorithms that allow computers to learn based on data. The model can be improved through learning (*e.g.,* by applying data to the model for "training" the algorithms). The data are thus often referred to as "training data" or "learning data." Examples of learning types may include supervised learning, unsupervised learning, semi-supervised learning, self-supervised learning, multi-instance learning, inductive learning, reinforcement learning, transudative learning, learning to learn, etc. The diagnosis engine may be trained with correlation between data including the plurality of impedances of the cervical tissues and the preterm birth risk. In an embodiment, the information provider 110 may be trained with correlation between the plurality of corresponding impedances, pregnancy stage, and the preterm birth risk. The diagnosis engine is organized into a taxonomy based on one or more outcomes of the model and is trained by applying training data to the model. The model is adjusted (*e.g.*, improved) based on how it responds to the training data. Multiple sets of training data may be applied to the same model so that the model may be repeatedly improved.

In an embodiment, the diagnosis engine may be trained with the supervised learning. Input data may include at least one of spectral impedances, pH level, metabolites, temperature, pregnancy stage, and human microbiome, and the result corresponding the preterm birth risk. The result may be, for example, one of high risk, medium risk, or low risk. Another example, the result may be numerical values of preterm birth risk. The input data may have been acquired from many patients. For example, the impedances may include impedances of cervical tissues of patients. The Input data may further comprise cervical tissue data of a patient, pregnancy stage, and other factors, etc. In an embodiment, the spectral impedances include impedances at different frequencies.

FIG. 1B illustrate a conceptual diagram illustrating an example of a diagnosis engine according to some embodiments of the present disclosure.

In an embodiment, the diagnosis engine may comprise plurality of classifiers, *e.g.,* AI classifiers or trained classifiers. The number of classifiers can range between one and M, where M is a positive integer (*e.g.,* 4). Each classifier can be a different type of classifier, or the same classifier trained in a different way. For example, each classifier can be trained with different learning data, or implemented with a different algorithm. For example, at least one of patient's information, age, spectral impedances, pH level, metabolites, temperature, pregnancy stage, and human microbiome, and the result corresponding the preterm birth risk for learning the classifier may be different. In an embodiment, the spectral impedances include impedances at different frequencies.

Examples of learning the classifier with different learning data are as follows and the learning data may be combined. The classifier may be trained with data of a mother who has a different pregnancy stage. The different pregnancy stage may be the first trimester (*e.g.,* one to twelve week), the second trimester (*e.g.,* thirteen to twenty-six week), or the third trimester (twenty-seven to the birth). The period can be adjusted. One classifier may be trained with data of a mother who has experienced the preterm birth or has not experienced the preterm birth. The learning data can be a mother's data as the pregnancy stage is developing because the impedance of cervical tissues would be different per the pregnancy stage.

In another embodiment, the classifier can include a different network. The classifiers may comprise individual classifiers (*e.g.,* first classifier or classifiers on a first stage) and a "meta" classifier (*e.g.,* second classifier or a classifier on a second stage). The inputs to the first stage classifiers can be all the same, or at least one inputs can be different to each classifier.

The "meta" classifier takes the outputs of each individual classifier, and performs a classification based on the results (outputs) from each. This can take advantage of the strengths of individual classifiers and produce the end result of the risk of preterm birth with higher accuracy than if only one classifier is used. Each classifier takes inputs, where each input is different, however each input may be the same input to all classifiers. Examples of inputs can be at least one of a patient's information (age, preterm birth experience, pregnancy stage, etc.), spectral impedances (impedances at different frequencies), pH level, metabolites, temperature and human microbiome. The patient information may be data obtained from a patient chart without measurement, or may be measured and entered into the chart. The chart can be stored on the system 100. The inputs can further include a patient's past data (*e.g.,* previously measured impedance, etc.) because a patient can have a checkup periodically. The "meta" classifier outputs the preterm birth risk based on outputs from the individual classifiers (*e.g.,* the first stage classifiers). The output preterm birth risk can be represented by a signal value, multiple values, or different groups, for example, high risk, medium risk and low risk. More or less groups are possible also. The "meta" classifier can also output a numerical value instead of groups, for example, a value between 0 and 100 that represents the percentage risk of preterm birth. The "meta" classifier can also output a number of numerical values.

The diagnosis engine described above is an example of the present disclosure, but the diagnosis engine is not limited under the above descriptions.

In an embodiment, the measuring device 105 transfers the data to the information provider 110. The information provider 110 may obtain impedances corresponding the cervical tissues based on the plurality of sinusoidal signals from the cervix 10.

In an embodiment, the measuring device 105 or the information provider 110 is configured to detect other data including at least one of force applied between a tip of the electrodes and the cervical tissues, spectral impedances, pH level of the cervix 10, metabolites of a patient, temperature of the patient, and human microbiome. The system 100 may receive force applied between a tip of the electrodes and the cervical tissues, spectral impedances, pH level, temperature, human microbiome from various devices. For example, the measuring device 105 can measure the force. Another device can measure and/or send at least one of spectral impedances, pH level of the cervix 10, metabolites of a patient, temperature of the patient, and human microbiome.

In an embodiment, electrical impedance (from engineering perspective) may be measured in ohms, and can have a "real" part being the resistance in units on ohms, and an "imaginary" part that is the reactance in the units of ohms. Either the reactance or resistance can be zero, and the value is still considered an impedance in ohms. The impedance has a unique magnitude and phase. In an embodiment, the spectral impedances include impedances at different frequencies.

FIGs. 2A to 2B illustrate an example of the measuring device 105 according to some embodiments of the present disclosure.

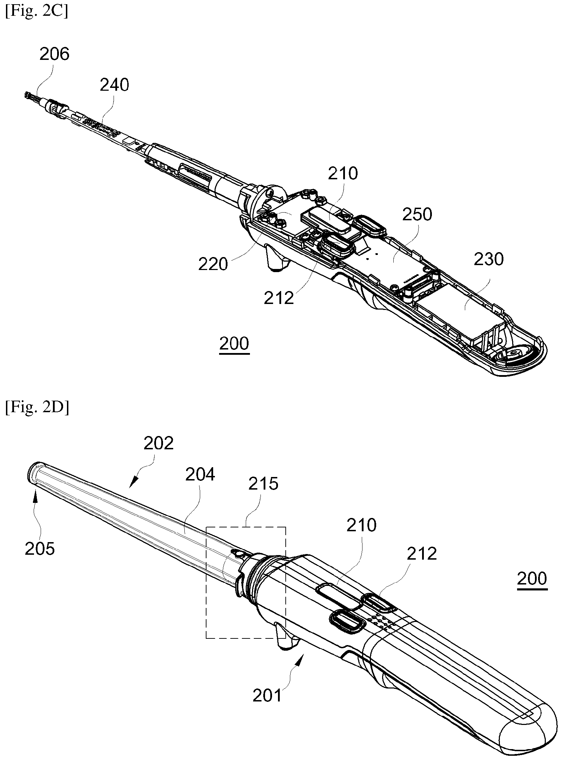

Referring to FIGs. 2A and 2C, the measuring device 105 may comprise a handle portion 201 and a probe tip portion 202 coupled to the handle portion 201. The probe tip portion 202 may be suitable for being introducing within a bodily opening provided in the mammalian body, for example, a vagina. The probe tip portion 202 may include a probe tip body 204.

The probe tip body 204 has an elongated-shape and protrudes from the handle portion 201. The probe tip body 204 may have an angled or curved elongated shape. For example, the shape of the probe tip body 204 may fit to the cervix.

The probe tip body 204 comprises two or more transmit electrodes (206, FIG. 2C) and two or more detect electrodes (206, FIG. 2C). The two or more transmit electrodes is designed to contact cervical tissues and apply a first plurality of sinusoidal signals at different frequencies to the cervical tissues. The two or more detect electrodes are designed to contact cervical tissues and detect a second plurality of sinusoidal signals generated in response to the first plurality of sinusoidal signals. The two or more transmit electrodes and the two or more detect electrodes may protrude from a tip of the probe tip body 204.

In an embodiment, two transmit electrodes can transmit signals that are 0, 90, 180 or 270 degrees out of phase. For example, if the second transmit signal is 180 degrees out of phase with the first transmit signal, this is equivalent to one being the negative of the other. This can also be applied for more than two transmit electrodes, where the transmit electrodes are treated as coupled pairs, again with one signal being 0, 90, 180 or 270 degrees out of phase with the other of the pair. Similarly, two receive electrodes can process the signals as 0, 90, 180 or 270 degrees out of phase with each other. This can also be applied for more than two receive electrodes, where the receive electrodes are treated as coupled pairs, again with one signal being 0, 90, 180 or 270 degrees out of phase with the other of the pair. The signal to be processed to derive the impedance can be derived by processing the signals separately, or the sum or derived from the difference of the two receive signals from the paired receive electrodes. After the sum or difference of the receive electrodes or processed separately, a substantial amount of the "noise" will cancel out from the received signals, allowing a more accurate impedance to be derived.

In an embodiment, the signals of the two paired transmit electrodes can be out of phase by a value other than 0, 90, 180 or 270 degrees, for example 45 degrees. In this case the signals received by the receive electrodes are processed by the same phase 45 in degrees.

In an embodiment, the received paired signals (*e.g.,* out of phase by 0, 90, 180 or 270 degrees), after being added or subtracted or processed separately, the value of the receive signal can be modulated (*e.g.,* multiplied) with a square wave, and then the values at the frequency or frequencies can be summed over time (*e.g.,* with an integrator). The summed signal is correlated to the value of the received signal. By knowing the transmitted signal and the received signal, the impedance at the frequency or at multiple frequencies can be calculated.

In an embodiment, the received paired signals (*e.g.,* out of phase by 0, 90, 180 or 270 degrees), after being added or subtracted or processed separately, the value of the receive signal can be at the frequency or frequencies can be extracted by using a spectrum analysis such as the Fourier transform, and then the values at the frequency or frequencies can be summed over time (*e.g.,* with an integrator). The summed signal is correlated to the value of the received signal. By knowing the transmitted signal and the received signal, the impedance at the frequency or at multiple frequencies can be calculated.

In an embodiment, the received paired signals (*e.g.,* out of phase by 0, 90, 180 or 270 degrees), after being added or subtracted or processed separately the value of the received signal can be at a single frequency, or multiple frequencies can be extracted by using a spectrum analysis such as the Fourier transform, and then the values at the frequency or frequencies can be processed individually at each frequency, such that the value of the signal at each frequency is calculated and processed independently of the signal at the other frequencies. Each signal at each frequency is correlated to the value of the received signal at the frequency. By knowing the transmitted signal and the received signal at the frequency, the impedance at the frequency can be calculated.

The handle portion 201 may comprise a button 212 and a display 210. The display 210 shows information to the user, for example, a force applied between the electrodes and the tissue. The display 210 may show the preterm birth risk, for example, by colors or numerical values. This can be through visual indicators (*e.g.,* LEDs), or using a graphical display. A user may start measurement with the button 212.

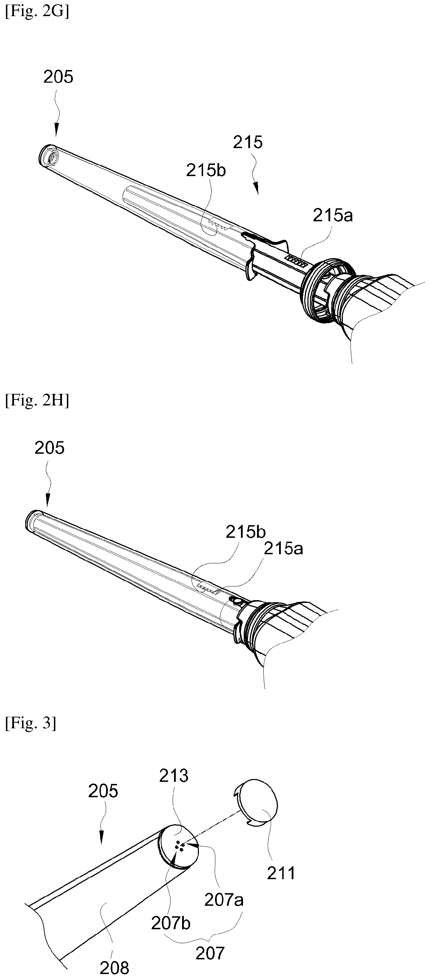

Referring to FIGs. 2B and 2D, in an embodiment, the measuring device 105 may further comprise a separate disposable tip 205. The disposable tip 205 can cover the probe tip body 204. An inner surface of the disposable tip 205 may tightly contact to an outer surface of the probe tip body 204. In an embodiment, a securing mechanism 215 (215, FIG. 2D) is configured to maintain the contact of the disposable tip 205 to the probe tip body 204, so that when the disposable tip 205 is pulled out from the bodily opening after measuring the impedance it will not separate from the rest of the probe. For example, one end portion of the disposable tip 205 is detachably coupled to the handle portion 201. A methodology of the securing mechanism 215 may include mechanical coupling of the disposable tip 205 and the handle portion 201, such as insertion, clamping, fastening, etc. For example, as illustrated in FIGs. 2G and 2H, the securing mechanism 215 may include the tongue 215b on the disposable tip 205, and a serration 215a on the probe tip body 204 so that as the disposable tip 205 is pulled toward handle the disposable tip 205 tightens and does not come undone.

In another embodiment, the securing mechanism 215 may can be achieved by magnetic elements or elastic material. For example, the handle portion 201 may pull the disposable tip 205 toward the handle portion by magnetism or by the elastic material, such as a spring.

The disposable tip 205 is configured to allow transmission and reception of electrical signals with reproducible accuracy. An electrical path between the two or more transmit electrodes 206 and the two or more detect electrodes 206 of the probe tip body 204, and the two or more transmit electrodes 207a (FIG. 3) and the two or more detect electrodes 207b (FIG. 3) of the disposable tip 205 is maintained using a contact mechanism for a reproducible impedance.

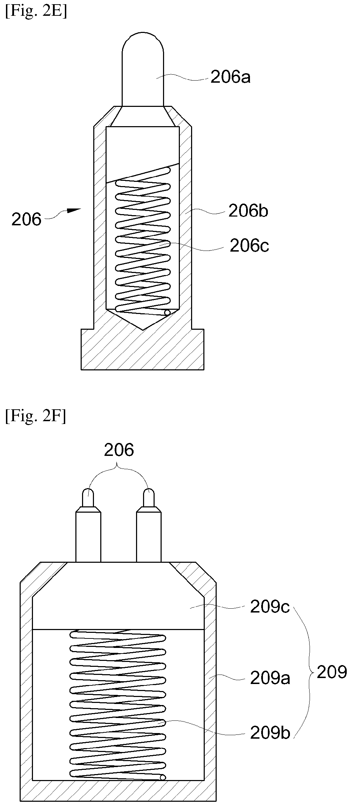

FIGs. 2E and 2F illustrate an example of the contact mechanism according to some embodiments of the present disclosure.

Referring to FIG. 2E, the contact mechanism may be established by the electrodes 206 of the probe tip body 204. In an embodiment, an individual electrode 206 may include an electrode tip 206a, electrode body 206b, and an elastic member 206c. The electrode body 206b may have a hollow shape such as cylindrical shape to accommodate the elastic member 206c therein, allowing it to move linearly. The elastic member 206c may be compressed by the electrode tip 206a when the disposable tip 205 covers the probe tip portion 202. Accordingly, the elastic member 206c may apply a force to push the linearly movable electrode tip 206 toward the electrodes 207 of the disposable tip 205. In an embodiment, the elastic member 206c may include a spring, a rubber, a structure which can be compressed and stretched, etc.

Referring to FIG. 2F, the contact mechanism 209 may include a base 209c, housing body 209a, and an elastic member 209b. The electrodes 206 is disposed on the base 209c. The housing body 209a may have a hollow shape such as cylindrical shape to accommodate the elastic member 209b therein. The elastic member 209b may be compressed by the base 209c when the disposable tip 205 covers the probe tip portion 202. Accordingly, the elastic member 209b may apply a force to push the linearly movable electrode tip 206 toward the electrodes 207 of the disposable tip 205. In an embodiment, the elastic member 209b may include a spring, a rubber, a structure which can be compressed and stretched, etc. The elastic member 209b may be replaced with a magnet.

The contact mechanism 209 can be used to give a good electrical contact from the two or more transmit electrodes 206 and the two or more detect electrodes 206 of the probe tip body 204 and the two or more transmit electrodes 207a (FIG. 3) and the two or more detect electrodes 207b (FIG. 3) of the disposable tip 205.

In an embodiment, the disposable tip 205 is designed to transmit electrical current to electrodes 207 in such a way that the impedance introduced in the electrodes 207 from the disposable tip 205 is substantially the same between different disposable tips, that is, the standard deviation of the incremental impedance added by the disposable tip is low.

Referring to FIG. 2C, the measuring device 105 may comprise a load cell assembly 220, a battery 230, and one or more Printed Circuit Boards (PCBs) 240, 250. One of the load cell assembly 220 and the PCBs 240, 250 may comprise a source, a controller (*e.g.,* a first controller, a second controller, etc.). The source is configured to generate the plurality of sinusoidal signals. The first controller is configured to control the source to provide the two transmit electrodes with the first sinusoidal signals at the different frequencies. The second controller is configured to control the detection of the second sinusoidal signals from the two or more detect electrodes at the different frequencies and to obtain a plurality of corresponding impedances of the cervical tissues based on the first and second sinusoidal signals. The measuring device 105 may further comprise a force calculator (not shown) for determining a force applied between a tip of the probe tip body and the cervical tissues. One of the load cell assembly 220 and the PCBs 240, 250 may further comprise a memory.

In an embodiment, the first sinusoidal signals are provided with different frequencies concurrently. For example, plural sinusoidal signals of different frequencies are overlapped and applied to the tissues concurrently. In an embodiment, the first sinusoidal signals are provided with different frequencies sequentially, where each set can contain one or more of a subset of the totality of sinusoidal signals (combinations of signals at different times).

In a further embodiment, a subset of the first sinusoidal signals are provided one frequency at a time, where all data quality criteria are applied and met before moving on to the second subset of different frequencies sequentially. For example, the first subset of sinusoidal signals of the first frequencies are transmitted, and after having met the data quality criteria, the first sinusoidal signals of the second subset of frequencies are transmitted.

FIG. 3 illustrates an example of the disposable tip 205 according to some embodiments of the present disclosure.

Referring to FIG. 3, the disposable tip 205 may comprise a probe cover 208, tip electrodes 207, and a tip cap 213. The disposable tip 205 may further includes an end cap 211 which covers the tip cap 213. The probe cover 208 covers the probe body 204. The tip electrodes 207 are disposed at a tip of the prove cover 208. The tip cap 213 is disposed at the probe cover 208 and covers the tip electrodes 207. The end cap 211 is disposed on the tip cap 213 and covers the tip cap 213 to prevent the tip electrodes 207 from being exposed outside when not used and make autocalibration. Thus, the end cap 211 may prevent erroneously and unintended contact of the tip electrode 207. The end cap 211 has an electric path (not illustrated) connecting the transmit electrodes and the receive electrodes 206 via the tip electrodes 207 when the end cap 211 is installed on the disposable tip 205. The impedance within the end cap 211 of each path between a transmit electrode and a receive electrode has a known fixed impedance (e.g., zero ohms, 5 ohms, 10 ohms, etc.). The impedances between the different sets of transmit electrodes and receive electrodes does not need to be the same, they can be unique for each set. The impedance between the different sets of transmit electrodes and receive electrodes can also be the same.

In an embodiment, since the disposable tip 205 is introduced within a bodily opening, the disposable tip 205 is waterproof such that bodily fluids cannot penetrate the disposable tip 205. The disposable tip can also be coated so that there is low friction between the disposable tip and the tissue of the patient during insertion.

The tip electrodes 207 comprises tip transmit electrodes 207a and tip detect electrodes 207b. The tip transmit electrodes and tip detect electrodes 207a, 207b are designed to contact to the cervical tissues. The tip transmit electrodes and tip detect electrodes 207a, 207b correspond to the transmit electrodes and the detect electrodes 206, respectively. The tip transmit electrodes and tip detect electrodes 207a, 207b electrically couple to the transmit electrodes and the detect electrodes 206, respectively. The numbers of the tip transmit electrodes and tip detect electrodes 207a, 207b may be same as those of the transmit electrodes and the detect electrodes 206.

In an embodiment, the disposable tip 205 may be introduced within the bodily opening. First sinusoidal signals may be applied to the cervical tissues through the tip transmit electrodes 207a. Second sinusoidal signals may be detected by tip detect electrodes 207b, and then transferred to the detect electrode 206 of the measuring device by electrical path between them.

After using the disposable tip 205, it is beneficial that the used disposable tip 205 is not used again for hygienic grounds. To prevent the re-use of the disposable tip 205, the present disclosure suggests the following attachment/detachment mechanisms, for example.

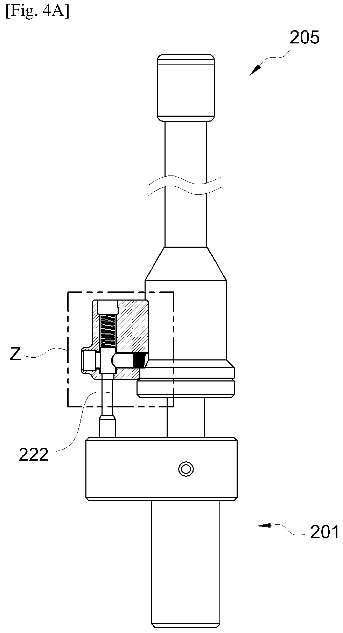

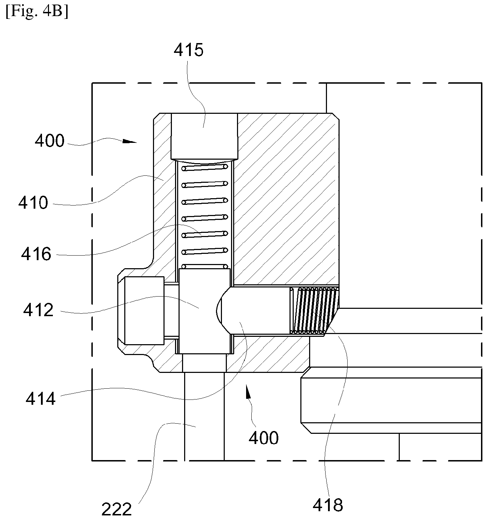

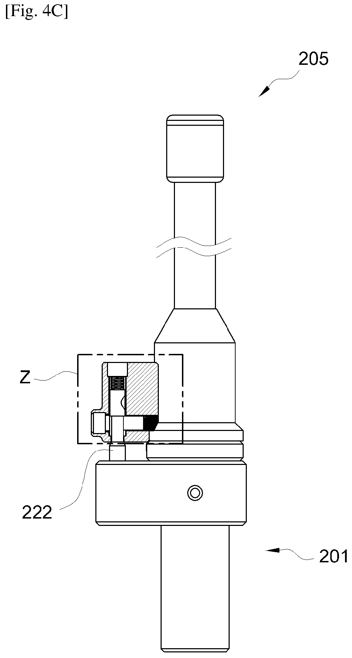

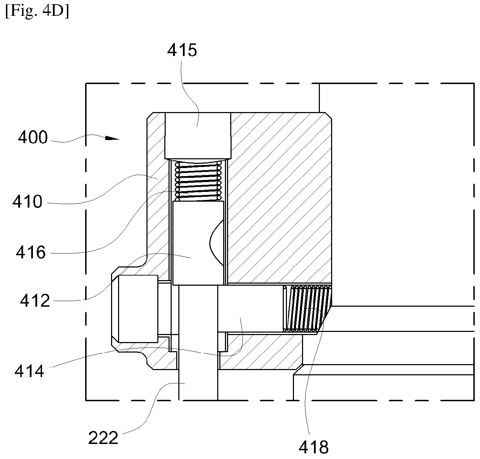

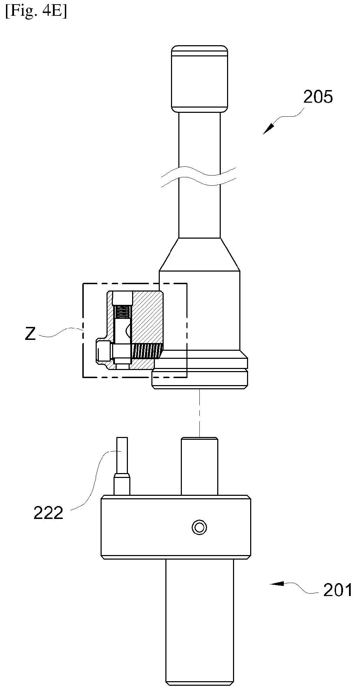

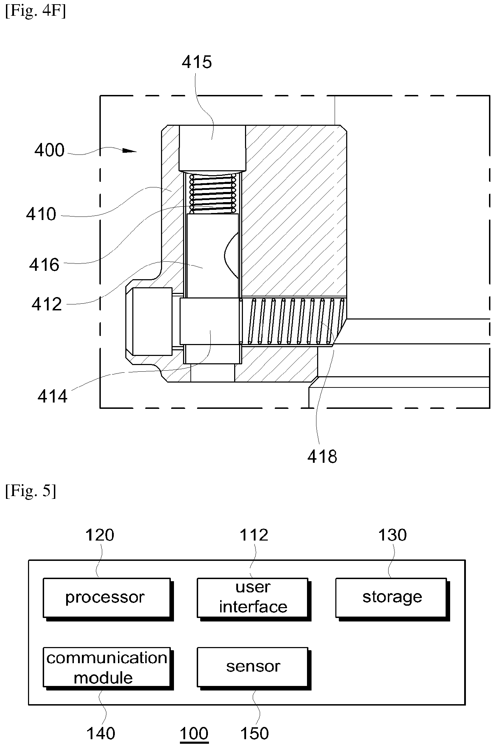

FIGs. 4A, 4C, and 4E illustrate attachment/detachment mechanisms of a disposable tip and a measuring device according to some embodiments of the present disclosure. FIGs. 4B, 4D, and 4F illustrate an enlarged view of the portion Z of FIGs. 4A, 4C, and 4E, respectively.

FIGs. 4A and 4B illustrate attachment/detachment mechanisms of a disposable tip and a measuring device before the disposable tip 205 is attached to the measuring device 105. Referring to FIGs. 4A and 4B, the handle portion 201 of the measuring device 105 comprises an insert protrusion 222, and the disposable tip 205 comprises a re-use prevention module 400. The insert protrusion 222 is configured to be inserted within the re-use prevention module 400 when the disposable tip 205 is coupled to the handle portion 201 of the measuring device 105. The re-use prevention module 400 comprises a housing 410, and components 412, 414, 415, 416, 418 disposed in the housing 410. The components 412, 414, 415, 416, 418 may include a first block 412, a second block 414, a base 415, a first elastic member 416, and a second elastic member 418.

The first elastic member 416 is disposed between the first block 412 and the base 415. The first block 412 may move along a first direction. The first elastic member 416 may generate attraction or repulsion to be applied to the first block 412. For example, the attraction or the repulsion generated by the first elastic member 416 transfers the first block 412 along the first direction. The second block 414 is disposed in the housing 410 and can move along a second direction overlapping with the first direction.

The second block 414 may move along the second direction. The second elastic member 418 may generate attraction or repulsion to be applied to the second block 414. For example, the attraction or the repulsion generated by the second elastic member 418 transfers the second block 414 along the second direction. The second block 414 may restrict the movement of the first block 412. For example, the second block 414 may maintain the position of the first block 412 by physically pressing the first block 412 or physically preventing the first block 412 from moving. As illustrated in FIG. 4B, the first block 412 has a groove and a portion of the second block 414 is inserted in the groove of first block 412 to prevent the first block 412 from moving. In another view, the first block 412 may maintain the position of the second block 414 by blocking a path where the second block moves, as illustrated in FIG. 4B. The second elastic member 418 is compressed and to generate a force pushing the second block 414. However, the first block 412 restricts the movement of the second block 414.

The first elastic member 416 and the second elastic member 418 may include a spring, an elastic material, a structure which can be compressed and stretched, etc. The first and the second member 416, 418 may be replaced with a magnet.

Before use of the disposable tip 205 or coupling the disposable tip 205 and the handle portion 201 of the measuring device 105, the second block 418 does not prevent the insert protrusion 222 from being inserted into the re-use prevention module 400.

In an embodiment, before use of the disposable tip 205 or coupling the disposable tip 205 and the handle portion 201, the first elastic member 416 and/or the second block 414 maintains the position of the first block 412. In an embodiment, before use of the disposable tip 205 or coupling the disposable tip 205 and the handle portion 201, the first elastic member 416 is stretched and generates the attraction to attract the first block 412 toward the base 415. However, since the second block 414 restricts the movement of the first block 412, the first block 412 can maintain the position which the insert protrusion 222 is inserted into.

FIGs. 4C and 4D illustrate attachment/detachment mechanisms of a disposable tip 205 and a measuring device 105 when the disposable tip is attached to the measuring device 105. Referring to FIGs. 4C and 4D, when the insert protrusion 222 is inserted within the re-use prevention module 400, the insert protrusion 222 have the first block 412 move toward the base 416 by physically moving the first block 412, for example, along the first direction. If the first elastic member 416 is stretched and generates the attraction to attract the first block 412, the first block 412 would be easily moved. As illustrated in FIG. 4D, even though the first block 412 moves and does not block the path of the second block 414, the second block 414 dose not move because the insert protrusion 222 replaces the position of the first block 412 and restricts the movement of the second block 414 during the coupling the disposable tip 205 and the handle portion 201.

In an embodiment, the first elastic member 416 may pull the first block 412 to keep the first block not blocking the path of the second block 414 after the disposable tip 205 is detached from the handle portion 201.

FIGs. 4E and 4F illustrate attachment/detachment mechanisms of a disposable tip 205 and a measuring device after the disposable tip is detached from the measuring device 105. Referring to FIGs. 4E and 4F, the second block 414 moves along the second direction when the insert protrusion 222 comes out of the re-use prevention module 400 and positions under the first block 412 to prevent the insert protrusion 222 from being inserted within the re-use prevention module 400 again.

In another embodiment, the methodology of preventing the re-use of the disposable tip 205 may include a code such as barcode, QR code, or a communicating method such as RFID, NFC, etc. For example, the system 100 may record the history of the use of the disposable tip 205 using the code on the disposable tip 205. For another example, the system may receive information about the use of the disposable tip 205 via wireless communication.

FIG. 5 illustrates a block diagram illustrating an apparatus or a system 100 for providing information for diagnosing preterm birth risk according to some embodiments of the present disclosure.

The system 100 comprises a processor 120, a user interface 112, storage 130, a communication module 140, and a sensor 150.

The processor 120 may include one or more processors such as a central processing unit (CPU), an application processor (AP) and/or a graphics processing unit (GPU), as well as digital memory, such as non-volatile memory (e.g., flash memory), both of which may be utilized to assist in the processing and storing of data. Data includes impedance obtained from the sensor 150, such as electrodes and accelerometers. Data further includes patient's personal information, patient's medical information including previous preterm birth history, pH level, metabolites, temperature, pregnancy stage, and human microbiome. The processor 120 is configured to analyze and process data. The processor 120 is configured to control overall operations of the system 100.

The storage 130 is configured to store the data above and correlation between the data and the preterm birth risk. The storage 130 is configured to store instructions that when executed, cause the processor 120 to perform certain actions. In an embodiment, when the instructions are executed, the instructions cause the processor to perform: applying a plurality of first sinusoidal signals at different frequencies to the cervical tissues; recording a plurality of second sinusoidal signals generated in response to the first sinusoidal signals in response to receiving the second sinusoidal signals;
computing a plurality of impedances of the cervical tissues based on the second sinusoidal signals; classifying the impedances into different groups, each representing different status, based on the correlation between the plurality of impedances and the preterm birth risk; and outputting information for diagnosing the preterm birth risk based on the classified groups.

The storage 130 is configured to store data and may include a digital data storage facility, which may be available through the internet or other networking configuration in a "cloud" resource configuration. In an embodiment, the storage 130 is configured to store codes for implementing the algorithms or models described above so that the diagnosis engine 110 can be established with a combination of the processor 120 and the codes.

The communication module 140 may be any of communications links, devices or apparatuses known to those skilled in the art by which information can be selectively communicated from an external input device to the system 100. The communication module 140 may include wireless communication techniques (e.g., RF and IR), wired communication techniques (e.g., electrical signals and optical signals), local area networks as well as embodying communication systems where communication is affected via the Internet.

The sensor 150 may be any of sensors known to those skilled in the art by which patient physical information (such as impedance of tissues) can be collected. For example, the sensor 150 may be part of the measuring device 105. In an embodiment, the sensor 150 may include an image sensor, a temperature sensor, a pressure sensor, an ultrasound sensor, a piezo sensor, electric signal sensor, etc. The sensor 150 may obtain images to show the cervix. The sensor 150 may measure a temperature of the tip of the disposable tip or the probe body 204 and a force applied between the electrodes 207 of disposable tip 205 (or electrodes 206 of the measuring device 105) and the cervical tissues. The sensor 150 may use the piezo sensor for confirming contact (or position) of the disposable tip (or the probe body 204) and target cervical tissues. The sensor 150 may send measured values or collected information to the processor 120 such that the processor 120 monitor the measured values or the collected information.

### Impedance and Cell Structure of Cervical Tissues

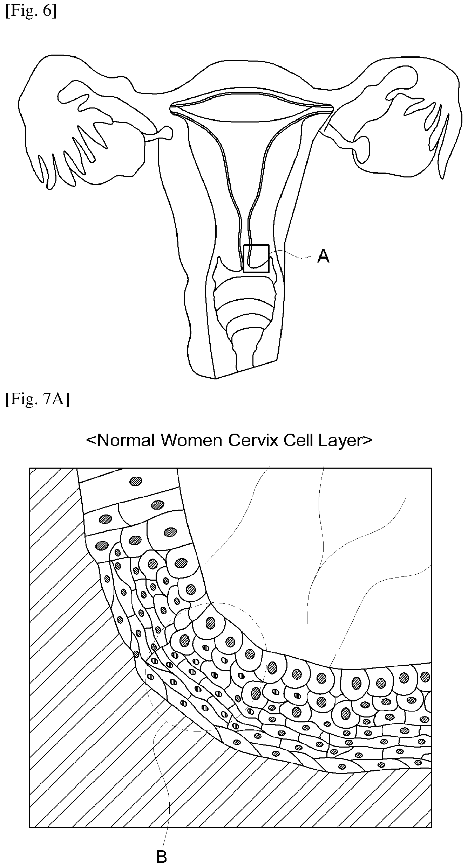

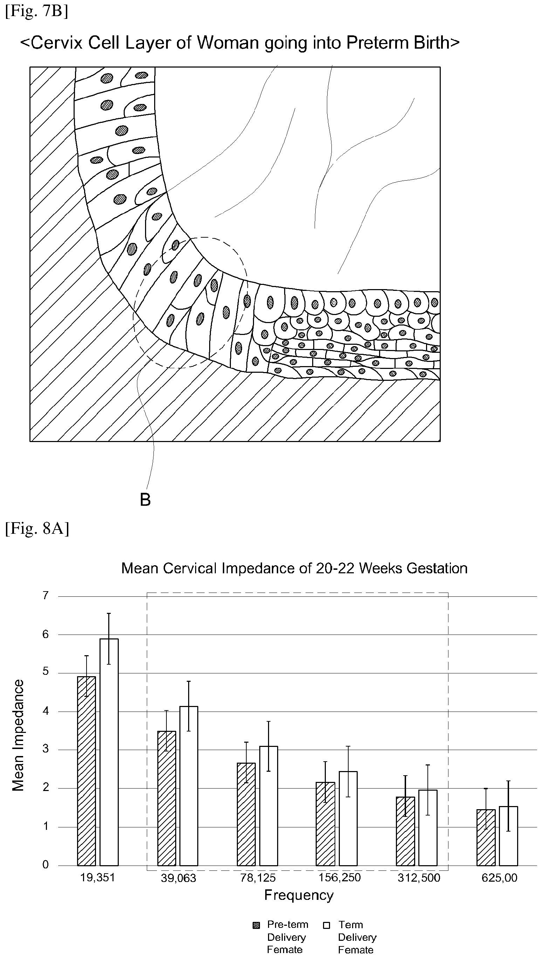

FIG. 6 illustrate an example of a cervix. FIGs. 7A and 7B illustrate an enlarged view of the portion A of FIG. 6. FIG. 7A shows a possible structure of the cervix at the pre-pregnancy stage or an early stage of pregnancy. FIG. 7B shows a possible structure of the cervix when nearing labor. Referring to FIGs. 7A and 7B, the cell structure or cell density on portion B is changed according to the pregnancy stage because an upper cell layer moves down. This movement of the cell layer causes impedance measured on the portion B to be changed based on when the impedance is measured. Thus, the present disclosure can predict the preterm birth by observing the cervical tissues, for example, measuring impedance of the cervical tissues.

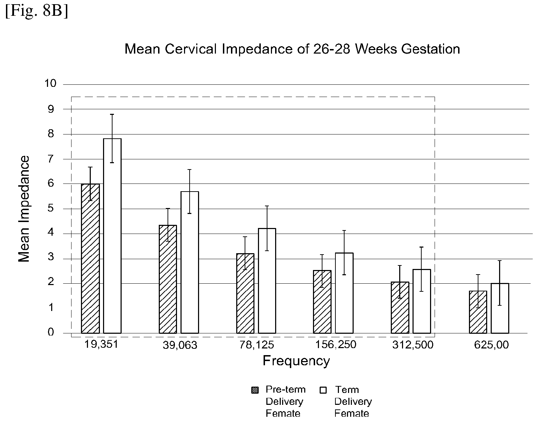

FIGs. 8A and 8B illustrate a graph of mean cervical impedances of mothers who experienced preterm delivery and term delivery. FIGs. 8A and 8B indicate that impedance of cervical tissues of mothers who experienced the preterm delivery is relatively different than that of mothers who experienced the term delivery across a frequency range.

### Impedance Measurement and Providing Information

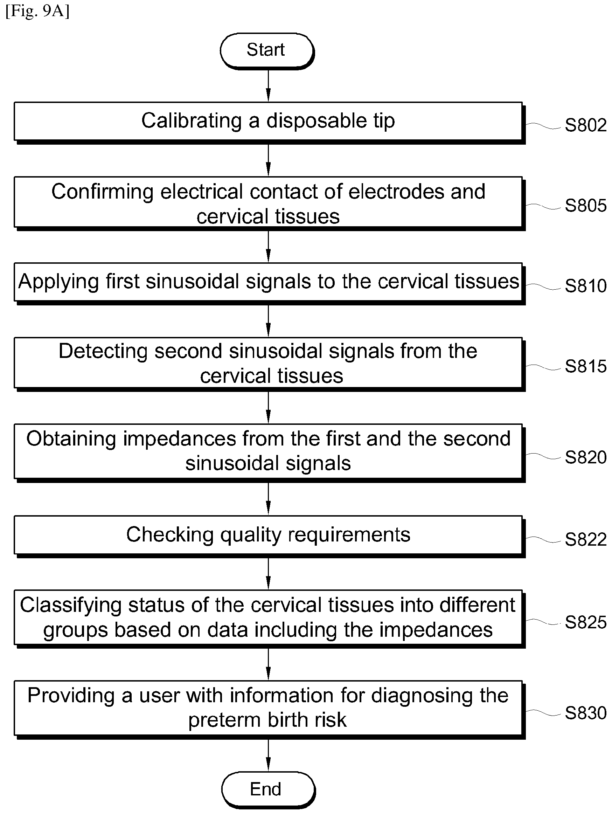

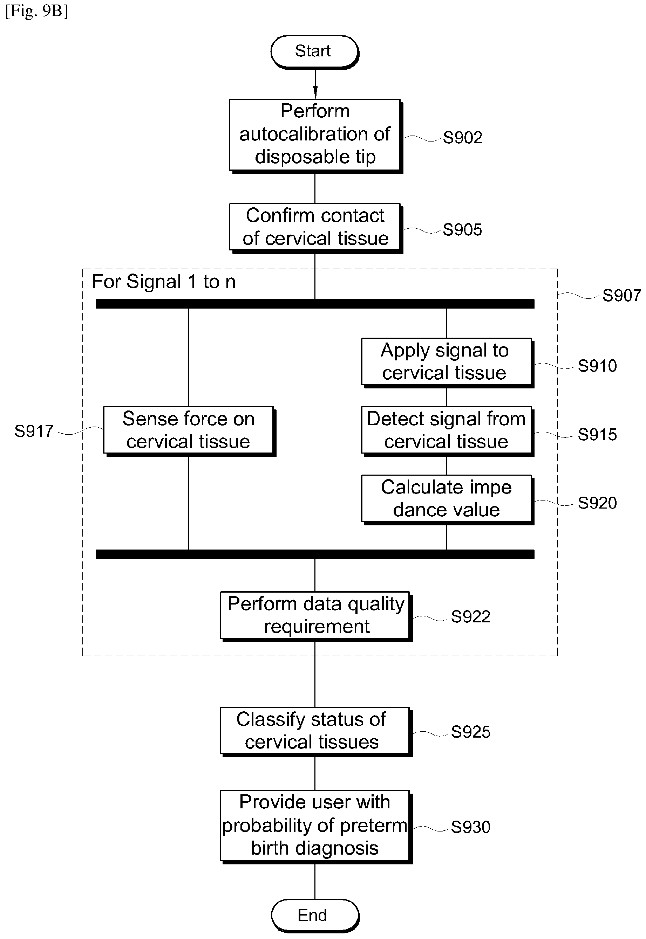

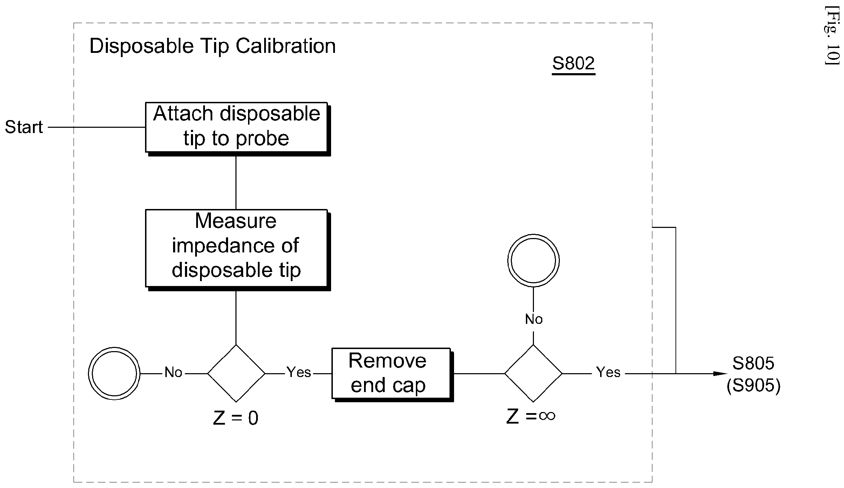

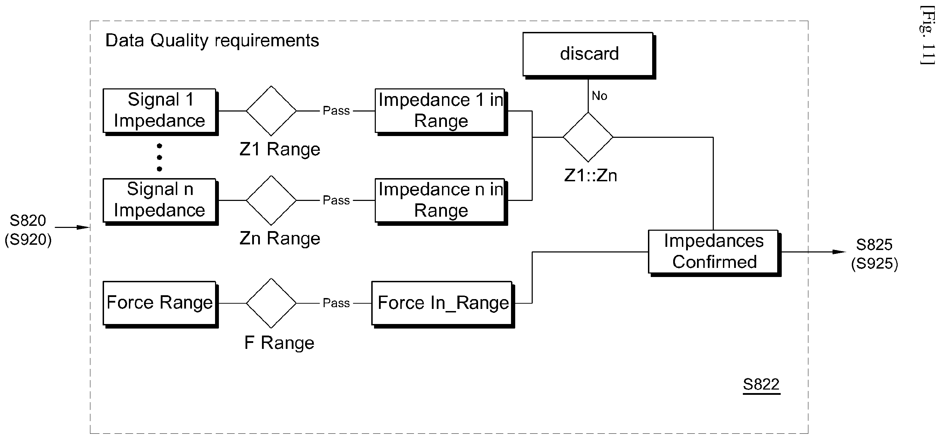

FIGs. 9A and 9B illustrate a flow-chart explaining a method of providing information for diagnosing preterm birth risk according to an embodiment of the present disclosure. FIG. 10 illustrates an example of calibrating a disposable tip according to an embodiment of the present disclosure. FIG. 11 illustrates an example of checking data quality requirements according to an embodiment of the present disclosure. The method may be implemented with the system 100. For example, a third controller of the system 100 may implement the checking data quality requirements.

Referring to FIG. 9B, blocks S902, S905, S910, S915, S920, S922, S925, and S930 are similar to or substantially same as blocks S802, S805, S810, S815, S820, S822, S825, and S980, respectively. Thus, repeated descriptions will be omitted.

In block 802, and referring to FIG. 10, the disposable tip 205 is calibrated. This occurs when the device is turned on and the disposable tip 205 has been installed, or after the device is turned on when the disposable tip 205 is then installed. The end cap 211 on the disposable tip 205 conducts electricity and imposes a known impedance between the transmit electrodes and the receive electrodes 206. For example, this impedance could be zero ohms. In another embodiment, the impedance may be a positive fixed impedance higher than zero ohms (*e.g.,* 5 ohms, 10 ohms, etc.). There may be also an independent electrical path between each set of transmit and receive electrodes. If there are 2 transmit and 2 receive electrodes, then there is 2 independent electrical paths.

If the end cap 211 is on the disposable tip 205, then system 100 calibrates the impedance path of the measuring device 105 to a known reference impedance. This can occur automatically or manually. This calibration is used until the disposable tip 205 is removed or the device is turned off. Then, the end cap 211 is removed. The impedance between the transmit electrodes and the receive electrodes 206 is indefinite after the end cap 211 is removed and before inserting into the patient. After the calibration, the measuring device 105 with the disposable tip 205 is ready for the next step. The disposable tip 205 and the end cap 211 are sterile.

In block S805, the system 100 may confirm an electric contact of electrodes 206 of the measuring device 105 and the cervical tissues of a mother. In an embodiment, a user (*e.g.,* doctor or nurse) may introduce the probe body 204 within a bodily opening to make the electric contact of electrodes 206 of the measuring device 105. The electrodes 206 may be electrically contact to the cervical tissues via electrodes 207 of the disposable tip 205. The system 100 may confirm an electric contact of electrodes 206 of the measuring device 105 and the cervical tissues with the sensor 150. The system 100 may confirm a position of the electrodes 206, 207 via the sensor 150.

In block S810, the first sinusoidal signals at different frequencies are applied to the cervical tissues. In an embodiment, the measuring device 105 may measure or calculate a force applied between the electrodes 207 of disposable tip 205 (or electrodes 206 of the measuring device 105) and the cervical tissues. In an embodiment, the measuring device 105 may measure or calculate the force with the sensor 150. In response to that the force reaches a selected (or predetermined) value or a selected (or predetermined) value range, the first sinusoidal signals at different frequencies are applied to the cervical tissues. The first sinusoidal signals at different frequencies are applied to the cervical tissues manually (*e.g.,* push a button) or automatically (*e.g.,* it is programmed).

In an embodiment, the force applied between the disposable tip 205 and the cervical tissues can be detected before at least one of the blocks S810, S815, S820. The measuring device 105 can analyze the second signals over entire periods of the transmission of the signals. The measuring device 105 is free to pick any signals when the force reaches the selected (or predetermined) value.

In block S815, the second sinusoidal signals are detected and recorded. The second sinusoidal signals correspond to the first sinusoidal signals after the signals have passed through the cervical tissue. For example, the first sinusoidal signals are injected into the cervical tissues, traversed through the cervical tissues, and then the traversed signals are detected as the second sinusoidal signals. The first sinusoidal signals are attenuated by the impedance of the cervical tissues to generate the second sinusoidal signals. The first sinusoidal signals may have a frequency as same as that of the second sinusoidal frequency but different amplitude and phase.

In an embodiment, in response to that the force applied between the electrodes 207 of disposable tip 205 (or electrodes 206 of the measuring device 105) and the cervical tissues reaches a selected (or predetermined) value or a selected (or predetermined) value range, the second sinusoidal signals are recorded from the cervical tissues. For example, the selected (or predetermined) value may be about 2 N (newton). For example, the selected (or predetermined) value range may be in a range of 1 N to 3 N. The second sinusoidal signals may be recorded manually (*e.g.,* push a button) or automatically (*e.g.,* it is programmed).

The system 100 may receive the second sinusoidal signals. In an embodiment, when the second sinusoidal signals are detected and recorded, the force applied between the electrodes 206 or 207 and the cervical tissues are also stored.

In block S820, impedances are obtained from the first and the second sinusoidal signals. The system 100 may compute the impedances from the first and the second sinusoidal signals. The impedances may be stored in the memory of the measuring device or storage 130.

In an embodiment, an impedance at one frequency can be calculated or measured multiple times, for example, 4, 5, 6, 8, 9 and 10 times. For the multiple measurement or calculation, at least one of the first signals, the second signal, the force can be obtained multiple times at the one frequency. From this multiple measurement or calculation, an overall impedance that is the average of many impedances can be obtained over time.

In an embodiment, the impedance at a corresponding frequency may be in selected (or predetermined) ohm range. For example, the selected (or predetermined) range may be in a range of 1 to 10 ohms.

In block S822, the system 100 checks the data quality requirements. Referring to FIG. 11, it determines that the calculated impedance for each signal in block S820 is in a selected (or predetermined) range. In an embodiment, the overall impedance can be used. In response to determining that the calculated impedance in block S820 is in a selected (or predetermined) range, the calculated impedance is stored in in the memory of the measuring device or storage 130. For example, a calculated impedance Z1 originated from signal 1 is stored in response to determining that the calculated impedance 1 is in a selected (or predetermined) range. Accordingly, the impedances Z1-Zn, which are determined in the selected (or predetermined) range, may be stored. The impedances Z1-Zn are compared with each other, and a ratio of the impedances Z1-Zn is calculated. For example, a ratio of the impedance Z1 and the impedance Zn is obtained. The ratio of impedances can be compared to determine if the ratio is greater than or equal to 1.0, or less than 1.0, and this can be used as further data quality criteria . The force of the disposable tip 205 on the cervical tissue can be monitored over time. It is determined that the force of the disposable tip 205 is in a selected (or predetermined) range. In response to determining that the force of the disposable tip 205 is in a selected (or predetermined) range, the force may be stored in the memory of the measuring device or storage 130 and correlated with the time that the corresponding impedance measurement was acquired. In an embodiment, it is determined that the force and the impedances (or the ratio of impedances) are in the selected (or predetermined) range, the impedances are used for the analysis for the risk of preterm birth.

In an embodiment, a sample impedance can be acquired at a particular time and the sample impedance can be used for calculating overall impedance at a particular frequency. That is to say, the sample impedance can contribute to the overall impedance that is the average of many impedances acquired over time.

In an embodiment, a calculated impedance value is checked for data quality requirements before being used to contribute to the analysis for the risk of preterm birth. This also includes the impedance values at more than one frequency. If the impedance value does not pass the data quality requirements, the impedance may be discarded. The data quality requirements include device performance, the value range of the impedance value in relation to the frequency at which the impedance value was generated, the impedance values monitored over time, and the force of the tip with the patient cervical tissue when the impedance was obtained. All data quality requirements are satisfied before using the impedance for preterm birth risk.

In an embodiment, the data quality requirements related to device performance include the software monitoring the electrical and electronic hardware devices during signal generation, signal reception, and impedance calculation. If any device selected (or required) for signal generation, signal reception, and impedance calculation has a hardware fault, or is performing outside of the specifications of the device, the impedance value calculated shall be discarded. This can include but is not limited to device communication errors, device overflow or underflow, out-of-bounds errors, clock errors, etc. If such a condition exists, the system may automatically correct the issue, or inform the user to correct the issue. After the issue has been resolved the impedance processing of the impedance values can continue.

In an embodiment, the data quality requirements related to the values of the impedance can include monitoring the value of the impedance with respect to the impedance amplitude (*e.g.,* defined as the square root of the sum of the real part of the impedance squared and the imaginary part of the impedance squared), the phase of the impedance, the real part of the impedance and/or the imaginary part of the impedance. One or more of these values can be monitored concurrently, and it is beneficial for all quality conditions on the values pass before the impedance can contribute to the calculation of the risk of preterm birth. The quality conditions for impedance can include, but are not limited to, the impedance must be within a specific range based on the frequency used to acquire the impedance. For example, at 100 Hz frequency, impedances are between 1 ohm and 100 ohms, while at 1000 Hz frequency, impedances are between 5 ohm and 50 ohms, etc.). The impedance value may also be within a range in relation to impedances at other frequencies. For example, the impedance at the 1000 Hz may be lower than the impedance at 100 Hz, etc. In some embodiments, it is beneficial for the impedance values over different frequencies to conform to a certain shape. For example, the average of impedances of frequencies within a lower frequency range may need to be higher in value than the average of impedances of frequencies within a higher frequency range.

In an embodiment, the data quality requirements related to impedance values monitored over time relate to the characteristics of the impedance value acquired over one or more time instances. These data quality requirements can include but are not limited to the amount of deviation over time, the time selected (or required) to "settle" at a value. The impedances can be monitored over a time period to determine if the deviations in the values over time are within a specified ohm range and/or monitored for the variance or standard deviation within a specified range. The first samples at each frequency or frequencies can also be monitored to determine when the impedance values have "stabilized", that is, if the startup reading fluctuations have decreased to acceptable levels. The monitoring over time can occur for one frequency, or multiple frequencies.

In an embodiment, the data quality requirements related to the force of the tip on the cervical tissue can be monitored over time and while the impedances are being calculated to determine when the tip force is within the selected (or required) range. If the tip force is not within the selected (or required) range, the impedances can be discarded for a select range of frequencies, or for all frequencies. When the tip force is within the acceptable range, and this has persisted for a minimum of a specified time period, the impedances can be used to contribute to preterm birth risk. The operator can be informed if the tip force is outside of the selected (or required) range, including information on if more or less force is selected (or required). This can be through visual indicators (*e.g.,* LEDs), or using a graphical display.

In another embodiment, the tip force can be controlled by a constant force mechanism such that the force being applied through the tip to the cervical tissue is within the ideal range. For example, the tip may allow for linear translation over a range of distances up to 5cm. Within the previously defined range, the tip will apply a constant output force, for example, 5N. The force may be adjusted per a user's choice or selection. A constant force mechanism may take the form of a flexure, cam, or spring system. The constant force mechanism may adopt a well-known art, for example, U.S. patent No. 5,649,454.

In an embodiment, simple contact sensor(s) may be employed to monitor the mechanism endpoint(s) to ensure that the tip displacement is within the constant force range. For example, when the mechanism reaches the end of the available travel, an electrical contact is made, which may alert the user to modulate the applied displacement. The contact sensor may also be monitored by the software to trigger data quality condition.

In another embodiment, the current tip force can be used control actuators that can affect the instantaneous force being applied by the tip on the cervical tissue. For example, if the tip force is too low, actuators can move the tip closer to the cervical tissue creating a higher force. This can continue until the tip force is within the selected (or required) range.

In another embodiment, the above requirements for device performance, the value range of the impedance value in relation to the frequency at which the impedance value was generated, the impedance values monitored over time, and the force of the tip with the patient cervical tissue when the impedance was obtained can be monitored over time to give an overall indication if the calculated impedance value(s) at that time have satisfied the data quality requirements. If they have satisfied the requirements, then there could be a condition that this must occur for a sum of a minimum time period to be considered an acceptable impedance sample that one or more frequencies. There can be a further condition that there must be more than one such acceptable impedance sample to create the selected (or required) conditions to be able to calculate the risk of preterm birth. For example, there could be a condition that there must be at least 10 acceptable impedance samples at each frequency to be able to generate the risk for preterm birth using system 100.

In another embodiment, other relevant data such as previous preterm birth history, pH level, metabolites, temperature, and human microbiome that has been collected can also be used to contribute to the risk assessment for preterm birth. It is beneficial for the data quality requirements for the pH level be within a certain pH range. It is beneficial for the data quality requirements for the metabolites be within a certain value range. It is beneficial for the data quality requirements for the temperature be within a certain temperature range. It is beneficial for the data quality requirements for the microbiome be within a certain value range. Each of these data can contribute to the risk assessment for preterm birth for each data value that has met the data quality requirements.

Referring to FIG. 10 and block S917, the tip force may be sensed during performing the S910(S810), S915(S815), and S920(S820). For example, when the sensed force is in a selected (or predetermined) range, it is determined that the S910(S810), S915(S815), and S920(S820) are performed correctly. The sensed force may be used for the data quality requirements in block S822 or S922. For example, the diagnosis engine 110 or the third controller can only use impedances for the data quality requirements when the force is in a selected (or predetermined) range. Or, for example, the diagnosis engine 110 or the third controller can only use signals detected when the force is in a selected (or predetermined) range for calculating impedance value in block S920.

Referring to FIG. 10 and block S907, the S910(S810), S915(S815), S920(S820), and S922(S822) may be performed for one signal, and the S910(S810), S915(S815), S920(S820), and S922(S822) may be repeated for each signal before S825 or S925. The repeat can be performed concurrently in the signal 1 to n, or sequentially from the signal 1 to n.

In block S825, the system 100 classifies the data values that met the data quality requirements and derived from the cervical tissues into different groups based on data including the impedances at different frequencies. The data may further comprise correlation between the impedances and the preterm birth risk. The data may further comprise at least one of force applied between a tip of the two transmit electrodes and the cervical tissues, spectral impedances, previous preterm birth history, pH level, metabolites, temperature, and human microbiome.

In block S830, the system 100 provides a user with information for diagnosing the preterm birth risk. The system 100 may notify a user of the information via the user interface 112. The user interface 112 may comprise a display, a LEDs, a speaker, etc.

In an embodiment, the storage 130 of the system 100 is configured to store instructions. When the instructions are executed by the processor 120, the instructions cause the processor to apply the first sinusoidal signals to the cervical tissues, receive the second sinusoidal signals, compute the impedances from the first and second sinusoidal signals and/or other data, classify status of the cervical tissues into different groups and provide a user with information for diagnosing the preterm birth risk.

The systems, methods, and devices described herein each have several aspects, no single one of which is solely responsible for its beneficial attributes. Without limiting the scope of this disclosure, several non-limiting features will now be discussed briefly. The following paragraphs describe various example methods. Corresponding devices, systems, and/or other hardware that performs some or all of the methods described in any particular example are also contemplated. A system of one or more computers can be configured to perform particular operations or actions by virtue of having software, firmware, hardware, or a combination installed in the system that in operation causes the system to perform the actions. One or more computer programs can be configured to perform particular operations or actions by virtue of including instructions that, when executed by data processing apparatus, cause the apparatus to perform the actions.

### Additional Considerations

Each of the processes, methods, and algorithms described herein and/or depicted in the attached figures may be embodied in, and fully or partially automated by, code modules executed by one or more physical computing systems, hardware computer processors, application-specific circuitry, and/or electronic hardware configured to execute specific and particular computer instructions. For example, computing systems can include general purpose computers (*e.g.,* servers) programmed with specific computer instructions or special purpose computers, special purpose circuitry, and so forth. A code module may be compiled and linked into an executable program, installed in a dynamic link library, or may be written in an interpreted programming language. In some implementations, particular operations and methods may be performed by circuitry that is specific to a given function.

Further, certain implementations of the functionality of the present disclosure are sufficiently mathematically, computationally, or technically complex that application-specific hardware or one or more physical computing devices (utilizing appropriate specialized executable instructions) may be necessary to perform the functionality, for example, due to the volume or complexity of the calculations involved or to provide results substantially in real-time. For example, a video may include many frames, with each frame having millions of pixels, and specifically programmed computer hardware may be beneficial to process the video data to provide an image processing task or application in a commercially reasonable amount of time.

Code modules or any type of data may be stored on any type of non-transitory computer-readable medium, such as physical computer storage including hard drives, solid state memory, random access memory (RAM), read only memory (ROM), optical disc, volatile or non-volatile storage, combinations of the same and/or the like. The methods and modules (or data) may also be transmitted as generated data signals (e.g., as part of a carrier wave or other analog or digital propagated signal) on a variety of computer-readable transmission mediums, including wireless-based and wired/ cable-based mediums, and may take a variety of forms (*e.g.,* as part of a single or multiplexed analog signal, or as multiple discrete digital packets or frames). The results of the disclosed processes or process steps may be stored, persistently or otherwise, in any type of non-transitory, tangible computer storage or may be communicated via a computer-readable transmission medium.

Any processes, blocks, states, steps, or functionalities in flow diagrams described herein and/or depicted in the attached figures should be understood as potentially representing code modules, segments, or portions of code which include one or more executable instructions for implementing specific functions (*e.g.,* logical or arithmetical) or steps in the process. The various processes, blocks, states, steps, or functionalities can be combined, rearranged, added to, deleted from, modified, or otherwise changed from the illustrative examples provided herein. In some embodiments, additional or different computing systems or code modules may perform some or all of the functionalities described herein. The methods and processes described herein are also not limited to any particular sequence, and the blocks, steps, or states relating thereto can be performed in other sequences that are appropriate, for example, in serial, in parallel, or in some other manner. Tasks or events may be added to or removed from the disclosed example embodiments. Moreover, the separation of various system components in the implementations described herein is for illustrative purposes and should not be understood as requiring such separation in all implementations. It should be understood that the described program components, methods, and systems can generally be integrated together in a single computer product or packaged into multiple computer products. Many implementation variations are possible.

The processes, methods, and systems may be implemented in a network (or distributed) computing environment. Network environments include enterprise-wide computer networks, intranets, local area networks (LAN), wide area networks (WAN), personal area networks (PAN), cloud computing networks, crowd-sourced computing networks, the Internet, and the World Wide Web. The network may be a wired or a wireless network or any other type of communication network.

The disclosure includes methods that may be performed using the subject devices. The methods may comprise the act of providing such a suitable device. Such provision may be performed by the end user. In other words, the "providing" act merely requires the end user obtain, access, approach, position, set-up, activate, power-up or otherwise act to provide the requisite device in the subject method. Methods recited herein may be carried out in any order of the recited events which is logically possible, as well as in the recited order of events.

The systems and methods of the disclosure each have several innovative aspects, no single one of which is solely responsible or selected (or required) for the beneficial attributes disclosed herein. The various features and processes described above may be used independently of one another, or may be combined in various ways. All possible combinations and subcombinations are intended to fall within the scope of this disclosure. Various modifications to the implementations described in this disclosure may be readily apparent to those skilled in the art, and the invention is defined by the wording of the claims.

Certain features that are described in this specification in the context of separate implementations also can be implemented in combination in a single implementation. Conversely, various features that are described in the context of a single implementation also can be implemented in multiple implementations separately or in any suitable subcombination. Moreover, although features may be described above as acting in certain combinations and even initially claimed as such, one or more features from a claimed combination can in some cases be excised from the combination, and the claimed combination may be directed to a subcombination or variation of a subcombination. No single feature or group of features is necessary or indispensable to each and every embodiment.

## Claims

1. A system (100) for providing information for diagnosing preterm birth risk, comprising:
two or more transmit electrodes (206), configured to contact cervical tissues, for applying a plurality of first signals at different frequencies to the cervical tissues;
two or more detect electrodes (206), configured to contact the cervical tissues, for detecting a plurality of second signals generated in response to the first signals, the second signals corresponding to the first signals, respectively;
a source configured to generate a plurality of signals;
a first controller configured to control the source to provide the two or more transmit electrodes (206) with the first signals at the different frequencies;
a second controller configured to:
control detection of the second signals from the two or more detect electrodes (206) at the different frequencies,
obtain a plurality of corresponding impedances of the cervical tissues based on the first and the second signals, and
apply data quality requirements for diagnosing the preterm birth risk, the data quality requirements including checking at least one of signal generation, signal reception, impedance calculation, quality of the first signals, quality of the second signals, and the impedances;
a sensor (150) for sensing a force applied between a tip of the two or more transmit electrodes (206) and the cervical tissues, and the data further comprises the force, and
wherein the sensor (150) further comprises an actuator configured to translate the tip either towards or away from the cervical tissues, and wherein the actuator is configured to control translation of the transmit electrodes (206) such that the force applied between the tip of the two or more transmit electrodes (206) and the cervical tissues is within a required range;
a diagnosis engine configured to:
receive data including impedances that satisfy the data quality requirements,
classify the data into different groups, each representing different status, based on correlation between the data, patient's information, and the preterm birth risk, and
output information for diagnosing the preterm birth risk; and
a user interface (112) for notifying a user of the information.

2. The system (100) of claim 1, wherein the first and second signals are voltages, or alternatively, wherein the first and second signals are currents, or,
wherein the first controller is configured to control the source to provide the two or more transmit electrodes (206) with the first signals with a combination of concurrent signals and sequential signals, or further alternatively,
wherein the first controller is configured to control the source to provide the two or more transmit electrodes (206) with the first signals at the different frequencies concurrently, and optionally wherein the first controller is configured to control the source to provide the two or more transmit electrodes (206) with the first signals 0, 90, 180, or 270 degrees out of phase with each other.

3. The system (100) of claim 1, wherein the first controller is configured to control the source to provide the two or more transmit electrodes (206) with the first signals at the different frequencies sequentially, and optionally wherein the first controller is configured to control the source to provide the two or more transmit electrodes (206) with the first signals 0, 90, 180, or 270 degrees out of phase with each other.

4. The system (100) of claim 1, wherein the user interface (112) informs the user of the sensed force, or alternatively,
wherein the sensor (150) is configured to sense the force during the detection of the second signals, and the second controller obtains the impedances of the cervical tissues based on the first signal and the second signal only when it is determined that the force is in a selected range.

5. The system (100) of claim 1, wherein the data quality requirements include checking if each of the corresponding impedances is in a selected range, or alternatively
wherein the data further comprises at least one of each of the impedances at the different frequencies, a patient's information including age and pregnancy stage, pH level, metabolites, temperature, and human microbiome, and optionally
wherein the data quality requirements include checking if the at least one of each of the impedances at the different frequencies, the patient's information, pH level, metabolites, temperature, and human microbiome are within a selected range.

6. The system (100) of claim 1, wherein the user interface (112) is configured to notify the information with numerical values, or alternatively,
wherein the user interface (112) comprises at least one or more of a display, a speaker, and a visual indicator including light emitting diodes, or further alternatively,
further comprising storage (130) configured to store the correlation between the data, the patient's information, and the preterm birth risk.

7. The system (100) of claim 1, further comprising a probe body (204) to which the transmit and detect electrodes (206) are attached, and a disposable tip (205), and wherein the disposable tip (205) contains a set of tip transmit and detect electrodes (207) that electrically couple to the transmit and detect electrodes (206) of the system (100) respectively when the disposable tip (205) is installed, and optionally
wherein the system (100) includes a contact mechanism (209) configured to maintain a contact of the transmit and detect electrodes (206) and the set of the tip transmit and detect electrodes (207) of the disposable tip (205), or, alternatively,
wherein the transmit electrodes (206) and the detect electrodes (206) are located at an end of the probe body (204) and the probe body (204) is configured to be introduced within a vagina such that the transmit electrodes (206) and the detect electrodes (206) contact the cervical tissues when the probe body (204) is introduced within the vagina, wherein the disposable tip (205) is configured to cover the portion of the probe body (204) that is inserted into the vagina, the transmit electrodes (206) and the detect electrodes (206) and to allow transmission and reception of the first and the second signals, and optionally
further comprising a securing mechanism (215) configured to couple to the disposable tip (205) and the probe body (204) such that the disposable tip (205) and the probe body (204) moves together, and further optionally
wherein the contact mechanism (209) includes an elastic material configured to push the transmit and detect electrodes (206) toward the set of the tip transmit and detect electrodes (207) of the disposable tip (205) when the disposable tip (205) is installed.

8. The system (100) of claim 1, wherein the diagnosis engine comprises a plurality of first classifiers such that each has as the input of at least one of impedances, a patient's information including age and pregnancy stage, pH level, metabolites, temperature, and human microbiome, and of which each classifier can be a different classifier or the same classifier configured in a different way, and a second classifier, with as input the outputs of each of the first classifiers, and output an indication of the preterm birth risk.

9. The system (100) of claim 1, wherein the first controller configured to control the source to provide the two or more transmit electrodes (206) with the first signals of same frequency repeatedly.

10. The system (100) of claim 1, wherein the data quality requirements including checking if the corresponding impedances are within a selected range based on a frequency used to acquire the corresponding impedances.

11. The system (100) of claim 1, wherein the data quality requirements including checking if the impedances over certain time period and determining if a deviation of the impedances is within a selected range.

## Patentansprüche

1. System (100) zur Bereitstellung von Informationen zur Diagnose des Risikos einer Frühgeburt, umfassend:
zwei oder mehr Übertragungselektroden (206), die dazu konfiguriert sind, Gebärmutterhalsgewebe zu kontaktieren, um eine Vielzahl von ersten Signalen bei unterschiedlichen Frequenzen an das Gebärmutterhalsgewebe anzuwenden;
zwei oder mehr Detektionselektroden (206), die dazu konfiguriert sind, das Gebärmutterhalsgewebe zu kontaktieren, um eine Vielzahl von zweiten Signalen, die als Reaktion auf die ersten Signale erzeugt werden, zu detektieren, wobei die zweiten Signale jeweils den ersten Signalen entsprechen;
eine Quelle, die dazu konfiguriert ist, eine Vielzahl von Signalen zu erzeugen;
eine erste Steuerung, die dazu konfiguriert ist, die Quelle zu steuern, um den zwei oder mehr Übertragungselektroden (206) die ersten Signale bei den unterschiedlichen Frequenzen bereitzustellen;
eine zweite Steuerung, die zu Folgendem konfiguriert ist:
Steuern von Detektion der zweiten Signale von den zwei oder mehr Detektionselektroden (206) bei den unterschiedlichen Frequenzen,
Erhalten einer Vielzahl von entsprechenden Impedanzen des Gebärmutterhalsgewebes basierend auf den ersten und den zweiten Signalen, und
Anwenden von Datenqualitätsanforderungen zur Diagnose des Risikos einer Frühgeburt, wobei die Datenqualitätsanforderungen Prüfen von zumindest einem von Signalerzeugung, Signalempfang, Impedanzberechnung, Qualität der ersten Signale, Qualität der zweiten Signale und den Impedanzen beinhalten;
einen Sensor (150) zum Erfassen einer Kraft, die zwischen einer Spitze der zwei oder mehr Übertragungselektroden (206) und dem Gebärmutterhalsgewebe angewendet wird, und wobei die Daten ferner die Kraft umfassen, und
wobei der Sensor (150) ferner einen Aktor umfasst, der dazu konfiguriert ist, die Spitze entweder zu oder weg von dem Gebärmutterhalsgewebe zu verschieben, und wobei der Aktor dazu konfiguriert ist, Verschiebung der Übertragungselektroden (206) zu steuern, sodass die Kraft, die zwischen der Spitze der zwei oder mehr Übertragungselektroden (206) und dem Gebärmutterhalsgewebe angewendet wird, innerhalb einer erforderlichen Spanne ist;
eine Diagnosemaschine, die zu Folgendem konfiguriert ist:
Empfangen von Daten, beinhaltend Impedanzen, welche die Datenqualitätsanforderungen erfüllen,
Klassifizieren der Daten in unterschiedliche Gruppen, die jeweils unterschiedlichen Status darstellen, basierend auf Korrelation zwischen den Daten, Patienteninformationen und dem Risiko einer Frühgeburt, und
Ausgeben von Informationen zur Diagnose des Risikos einer Frühgeburt; und
eine Benutzerschnittstelle (112) zum Benachrichtigen eines Benutzers über die Informationen.

2. System (100) nach Anspruch 1, wobei die ersten und zweiten Signale Spannungen sind, oder alternativ wobei die ersten und zweiten Signale Ströme sind, oder
wobei die erste Steuerung dazu konfiguriert ist, die Quelle zu steuern, um den zwei oder mehr Übertragungselektroden (206) die ersten Signale mit einer Kombination von gleichzeitigen Signalen und sequentiellen Signalen bereitzustellen, oder ferner alternativ
wobei die erste Steuerung dazu konfiguriert ist, die Quelle zu steuern, um den zwei oder mehr Übertragungselektroden (206) die ersten Signale bei den unterschiedlichen Frequenzen gleichzeitig bereitzustellen, und wobei optional die erste Steuerung dazu konfiguriert ist, die Quelle zu steuern, um den zwei oder mehr Übertragungselektroden (206) die ersten Signale 0, 90, 180 oder 270 Grad phasenverschoben zueinander bereitzustellen.

3. System (100) nach Anspruch 1, wobei die erste Steuerung dazu konfiguriert ist, die Quelle zu steuern, um den zwei oder mehr Übertragungselektroden (206) die ersten Signale bei den unterschiedlichen Frequenzen sequentiell bereitzustellen, und wobei optional die erste Steuerung dazu konfiguriert ist, die Quelle zu steuern, um den zwei oder mehr Übertragungselektroden (206) die ersten Signale 0, 90, 180 oder 270 Grad phasenverschoben zueinander bereitzustellen.

4. System (100) nach Anspruch 1, wobei die Benutzerschnittstelle (112) den Benutzer über die erfasste Kraft informiert, oder alternativ
wobei der Sensor (150) dazu konfiguriert ist, die Kraft während der Detektion der zweiten Signale zu erfassen und die zweite Steuerung die Impedanzen des Gebärmutterhalsgewebes basierend auf dem ersten Signal und dem zweiten Signal nur erhält, wenn bestimmt wird, dass die Kraft in einer ausgewählten Spanne ist.

5. System (100) nach Anspruch 1, wobei die Datenqualitätsanforderungen Prüfen beinhalten, ob jede von den entsprechenden Impedanzen in einer ausgewählten Spanne ist, oder alternativ
wobei die Daten ferner zumindest eines von jedem von den Impedanzen bei den unterschiedlichen Frequenzen, Patienteninformationen, beinhaltend Alter und Schwangerschaftsstadium, pH-Niveau, Metaboliten, Temperatur und menschliches Mikrobiom umfassen, und optional
wobei die Datenqualitätsanforderungen Prüfen beinhalten, ob das zumindest eine von jedem von den Impedanzen bei den unterschiedlichen Frequenzen, den Patienteninformationen, pH-Niveau, Metaboliten, Temperatur und menschlichem Mikrobiom innerhalb einer ausgewählten Spanne sind.

6. System (100) nach Anspruch 1, wobei die Benutzerschnittstelle (112) dazu konfiguriert ist, die Informationen mit numerischen Werten zu benachrichtigen, oder alternativ
wobei die Benutzerschnittstelle (112) zumindest eines oder mehrere von einer Anzeige, einem Lautsprecher und einem visuellen Indikator, der Leuchtdioden beinhaltet, umfasst, oder ferner alternativ
ferner umfassend Speicher (130), der dazu konfiguriert ist, die Korrelation zwischen den Daten, den Patienteninformationen und dem Risiko einer Frühgeburt zu speichern.

7. System (100) nach Anspruch 1, ferner umfassend einen Sondenkörper (204), an dem die Übertragungs- und Detektionselektroden (206) angebracht sind, und eine Einwegspitze (205), und wobei die Einwegspitze (205) einen Satz von Spitzen-Übertragungs- und Detektionselektroden (207) enthält, die jeweils elektrisch an die Übertragungs- und Detektionselektroden (206) des Systems (100) koppeln, wenn die Einwegspitze (205) installiert ist, und optional
wobei das System (100) einen Kontaktmechanismus (209) beinhaltet, der dazu konfiguriert ist, einen Kontakt der Übertragungs- und Detektionselektroden (206) und des Satzes von den Spitzen-Übertragungs- und Detektionselektroden (207) der Einwegspitze (205) aufrechtzuerhalten, oder alternativ
wobei sich die Übertragungselektroden (206) und die Detektionselektroden (206) an einem Ende des Sondenkörpers (204) befinden und der Sondenkörper (204) dazu konfiguriert ist, innerhalb einer Vagina eingeführt zu werden, sodass die Übertragungselektroden (206) und die Detektionselektroden (206) das Gebärmutterhalsgewebe kontaktieren, wenn der Sondenkörper (204) innerhalb der Vagina eingeführt ist, wobei die Einwegspitze (205) dazu konfiguriert ist, den Abschnitt des Sondenkörpers (204), der in die Vagina eingesetzt wird, die Übertragungselektroden (206) und die Detektionselektroden (206) zu bedecken und Übertragung und Empfang der ersten und der zweiten Signale zu ermöglichen, und optional
ferner umfassend einen Sicherungsmechanismus (215), der dazu konfiguriert ist, an die Einwegspitze (205) und den Sondenkörper (204) zu koppeln, sodass sich die Einwegspitze (205) und der Sondenkörper (204) zusammen bewegen, und ferner optional
wobei der Kontaktmechanismus (209) ein elastisches Material beinhaltet, das dazu konfiguriert ist, die Übertragungs- und Detektionselektroden (206) zu dem Satz von den Spitzen-Übertragungs- und Detektionselektroden (207) der Einwegspitze (205) zu drücken, wenn die Einwegspitze (205) installiert ist.

8. System (100) nach Anspruch 1, wobei die Diagnosemaschine eine Vielzahl von ersten Klassifikatoren, sodass jeder als die Eingabe zumindest eines von Impedanzen, Patienteninformationen, beinhaltend Alter und Schwangerschaftsstadium, pH-Niveau, Metaboliten, Temperatur und menschliches Mikrobiom aufweist, und wobei jeder Klassifikator ein unterschiedlicher Klassifikator oder der gleiche Klassifikator sein kann, der auf eine unterschiedliche Weise konfiguriert ist, und einen zweiten Klassifikator, mit als Eingabe den Ausgaben von jedem von den ersten Klassifikatoren und Ausgabe einer Indikation des Risikos einer Frühgeburt umfasst.

9. System (100) nach Anspruch 1, wobei die erste Steuerung dazu konfiguriert ist, die Quelle zu steuern, um den zwei oder mehr Übertragungselektroden (206) die ersten Signale von gleicher Frequenz wiederholt bereitzustellen.

10. System (100) nach Anspruch 1, wobei die Datenqualitätsanforderungen Prüfen, ob die entsprechenden Impedanzen innerhalb einer ausgewählten Spanne sind, basierend auf einer Frequenz, die verwendet wird, um die entsprechenden Impedanzen zu erwerben, beinhalten.

11. System (100) nach Anspruch 1, wobei die Datenqualitätsanforderungen Prüfen, ob die Impedanzen über bestimmten Zeitraum und Bestimmen, ob eine Abweichung der Impedanzen innerhalb einer ausgewählten Spanne ist, beinhalten.

## Revendications

1. Système (100) de fourniture d'informations pour diagnostiquer un risque de naissance prématurée, comprenant :
deux électrodes de transmission ou plus (206), conçues pour entrer en contact avec les tissus du col de l'utérus, destinées à appliquer une pluralité de premiers signaux à des fréquences différentes aux tissus du col de l'utérus ;
deux électrodes de détection ou plus (206), conçues pour entrer en contact avec les tissus du col de l'utérus, destinées à détecter une pluralité de seconds signaux générés en réponse aux premiers signaux, les seconds signaux correspondant aux premiers signaux, respectivement ;
une source conçue pour générer une pluralité de signaux ;
un premier dispositif de commande configuré pour commander à la source de fournir aux deux électrodes de transmission ou plus (206) les premiers signaux aux différentes fréquences ;
un second dispositif de commande configuré pour :
commander la détection des seconds signaux provenant des deux électrodes de détection ou plus (206) aux fréquences différentes,
obtenir une pluralité d'impédances correspondantes des tissus du col de l'utérus sur la base des premier et second signaux, et
appliquer des exigences de qualité de données pour diagnostiquer le risque de naissance prématurée, les exigences de qualité de données comprenant la vérification d'au moins un élément parmi la génération de signaux, la réception de signaux, le calcul d'impédances, la qualité des premiers signaux, la qualité des seconds signaux et des impédances ;
un capteur (150) destiné à détecter une force appliquée entre une pointe des deux électrodes de transmission ou plus (206) et les tissus du col de l'utérus, et les données comprennent en outre la force, et
ledit capteur (150) comprenant en outre un actionneur conçu pour translater la pointe en direction ou à l'opposé des tissus du col de l'utérus, et ledit actionneur étant conçu pour commander la translation des électrodes de transmission (206) de sorte que la force appliquée entre la pointe des deux électrodes de transmission ou plus (206) et les tissus du col de l'utérus se situe dans une plage requise ;
un moteur de diagnostic configuré pour :
recevoir des données comprenant des impédances qui satisfont aux exigences de qualité de données,
classer les données en différents groupes, chacun représentant un statut différent, en fonction de la corrélation entre les données, les informations de la patiente et le risque de naissance prématurée, et
délivrer des informations pour diagnostiquer le risque de naissance prématurée ; et
une interface utilisateur (112) pour notifier à un utilisateur les informations.

2. Système (100) selon la revendication 1, lesdits premier et second signaux étant des tensions, ou en variante, lesdits premier et second signaux étant des courants, ou,
ledit premier dispositif de commande étant configuré pour commander à la source de fournir aux deux électrodes de transmission ou plus (206) les premiers signaux avec une combinaison de signaux concurrents et de signaux séquentiels, ou en outre en variante,
ledit premier dispositif de commande étant configuré pour commander à la source de fournir aux deux électrodes de transmission ou plus (206) les premiers signaux aux différentes fréquences simultanément, et éventuellement ledit premier dispositif de commande étant configuré pour commander à la source de fournir aux deux électrodes de transmission ou plus (206) les premiers signaux à décalage de phase de 0, 90, 180 ou 270 degrés les uns par rapport aux autres.

3. Système (100) selon la revendication 1, ledit premier dispositif de commande étant configuré pour commander à la source de fournir aux deux électrodes de transmission ou plus (206) les premiers signaux aux différentes fréquences séquentiellement, et éventuellement ledit premier dispositif de commande étant configuré pour commander à la source de fournir aux deux électrodes de transmission ou plus (206) les premiers signaux à décalage de phase de 0, 90, 180 ou 270 degrés les uns par rapport aux autres.

4. Système (100) selon la revendication 1, ladite interface utilisateur (112) informant l'utilisateur de la force détectée, ou en variante,
ledit capteur (150) étant configuré pour détecter la force pendant la détection des seconds signaux, et ledit second dispositif de commande obtenant les impédances des tissus du col de l'utérus sur la base du premier signal et du second signal uniquement lorsqu'il est déterminé que la force se trouve dans une plage sélectionnée.

5. Système (100) selon la revendication 1, lesdites exigences de qualité de données comprenant la vérification pour savoir si chacune des impédances correspondantes se trouve dans une plage sélectionnée, ou en variante
lesdites données comprenant en outre au moins un élément parmi chacune des impédances aux différentes fréquences, les informations d'une patiente comprenant l'âge et le stade de grossesse, le niveau de pH, les métabolites, la température et le microbiome humain, et éventuellement
lesdites exigences de qualité de données comprenant la vérification pour savoir si ledit au moins un élément parmi chacune des impédances aux différentes fréquences, les informations de la patient, le niveau de pH, les métabolites, la température et le microbiome humain se trouvent dans une plage sélectionnée.

6. Système (100) selon la revendication 1, ladite interface utilisateur (112) étant configurée pour notifier les informations avec des valeurs numériques, ou en variante,
ladite interface utilisateur (112) comprenant au moins un ou plusieurs éléments parmi un dispositif d'affichage, un haut-parleur et un indicateur visuel comprenant des diodes électroluminescentes, ou en outre en variante,
comprenant en outre un dispositif de stockage (130) configuré pour stocker la corrélation entre les données, les informations de la patiente et le risque de naissance prématurée.

7. Système (100) selon la revendication 1, comprenant en outre un corps de sonde (204) auquel les électrodes de transmission et de détection (206) sont fixées, et une pointe jetable (205), et ladite pointe jetable (205) contenant un ensemble d'électrodes de transmission et de détection de pointe (207) qui sont électriquement couplées aux électrodes de transmission et de détection (206) du système (100) respectivement lorsque la pointe jetable (205) est installée, et éventuellement
ledit système (100) comprenant un mécanisme de contact (209) conçu pour maintenir un contact entre les électrodes de transmission et de détection (206) et l'ensemble des électrodes de transmission et de détection de pointe (207) de la pointe jetable (205), ou, en variante,
lesdites électrodes de transmission (206) et lesdites électrodes de détection (206) étant situées au niveau d'une extrémité du corps de sonde (204) et ledit corps de sonde (204) étant conçu pour être introduit à l'intérieur d'un vagin de sorte que les électrodes de transmission (206) et les électrodes de détection (206) entrent en contact avec les tissus du col de l'utérus lorsque le corps de sonde (204) est introduit à l'intérieur du vagin, ladite pointe jetable (205) étant conçue pour couvrir la partie du corps de sonde (204) qui est insérée dans le vagin, les électrodes de transmission (206) et les électrodes de détection (206) et pour permettre la transmission et la réception des premier et second signaux, et éventuellement
comprenant en outre un mécanisme de fixation (215) conçu pour se coupler à la pointe jetable (205) et au corps de sonde (204) de sorte que la pointe jetable (205) et le corps de sonde (204) se déplacent ensemble, et en outre éventuellement
ledit mécanisme de contact (209) comprenant un matériau élastique conçu pour pousser les électrodes de transmission et de détection (206) en direction de l'ensemble des électrodes de transmission et de détection de pointe (207) de la pointe jetable (205) lorsque la pointe jetable (205) est installée.

8. Système (100) selon la revendication 1, ledit moteur de diagnostic comprenant une pluralité de premiers classificateurs tels que chacun ait comme entrée au moins un élément parmi les impédances, les informations d'une patiente comprenant l'âge et le stade de grossesse, le niveau de pH, les métabolites, la température et le microbiome humain, et dont chaque classificateur peut être un classificateur différent ou le même classificateur configuré d'une manière différente, et un second classificateur, avec comme entrée les sorties de chacun des premiers classificateurs, et fournit une indication du risque de naissance prématurée.

9. Système (100) selon la revendication 1, ledit premier dispositif de commande étant configuré pour commander à la source de fournir aux deux électrodes de transmission ou plus (206) les premiers signaux de même fréquence à plusieurs reprises.

10. Système (100) selon la revendication 1, lesdites exigences de qualité de données comprenant la vérification pour savoir si les impédances correspondantes se trouvent à l'intérieur d'une plage sélectionnée sur la base d'une fréquence utilisée pour acquérir les impédances correspondantes.

11. Système (100) selon la revendication 1, lesdites exigences de qualité de données comprenant la vérification pour savoir si les impédances sur une certaine période de temps et la détermination pour savoir si une déviation des impédances se trouve dans une plage sélectionnée.
